# EUROPÄISCHE PATENTSCHRIFT

(11) **EP 1 751 684 B1**
(45) Veröffentlichungstag und Bekanntmachung des Hinweises auf die Patenterteilung: **31.07.2013**
(21) Anmeldenummer: 05748183.0
(22) Anmeldetag: 01.06.2005
(51) Int. Cl.: G01N 33/574

(54) **VERFAHREN ZUR ANWENDUNG VON AUF MARKERWERTEN BASIERENDEN DICHTEKARTEN BEI DER DIAGNOSE VON PATIENTEN MIT KRANKHEITEN INSBESONDERE VON TUMOREN**
METHOD FOR THE USE OF DENSITY MAPS BASED ON MARKER VALUES IN ORDER TO DIAGNOSE PATIENTS WITH DISEASES, PARTICULARLY TUMORS
PROCEDE D'APPLICATION DE CARTES DE DENSITES BASEES SUR DES VALEURS DE MARQUEURS LORS DU DIAGNOSTIC DE PATIENTS SOUFFRANT DE MALADIES, NOTAMMENT DE TUMEURS

(30) Priorität: 04.06.2004 DE 102004027429
(43) Veröffentlichungstag der Anmeldung: 14.02.2007
(73) Patentinhaber: Siemens Healthcare Diagnostics GmbH, 65760 Eschborn (DE)
(72) Erfinder: BUTZ, Hermann, 35423 Lich (DE); KELLER, Thomas, 04275 Leipzig (DE)
(74) Vertreter: Maier, Daniel Oliver
(86) Internationale Anmeldenummer: PCT/EP2005/005877
(87) Internationale Veröffentlichungsnummer: WO 2005/119564

(56) Entgegenhaltungen:
- WO-A-02/095650
- US-A- 5 501 983
- US-A1- 2003 233 197

## Beschreibung

Die Erfindung betrifft ein Verfahren zur Anwendung von auf Tumormarkerwerten, anderen Indikatorstoffen und -werten basierenden Dichtekarten bei der Diagnose von Patienten mit einer Tumorerkrankung, insbesondere dem Prostatakarzinom. Die Erfindung betrifft insbesondere die Anwendung eines mathematischen Verfahrens, mit dessen Hilfe anhand von Tumormarkerwertepaaren einem Patienten ein Risiko, an einem Tumor erkrankt zu sein, zugeordnet werden kann.

Aufgabe der Erfindung ist es, auf der Basis von für den Patienten gemessenen Tumormarkerwerten bzw. anderen Indikatorstoffen und -werten anhand einer Dichtekarte dem Patienten ein Risiko, ausgedrückt durch den positiven Vorhersagewert für eine spezifische klinische Anwendungssituation, zuzuordnen, an einem Tumor, insbesondere einem Prostatakarzinom erkrankt zu sein.

Bisher wird dem Stand der Technik nach die Diagnose von Tumoren, insbesondere dem Prostatakarzinom, anhand von Tumormarkern und anderen Indikatorstoffen und -werten in folgender Weise vorgenommen:
a) ein Indikatorstoff wird gemessen, wobei die Höhe des Indikatorstoffes mit dem Vorhandensein einer Tumorerkrankung korreliert. Unter Zuhilfenahme eines an einer Studienpopulation ermittelten Cut-Off-Wertes wird je nach Lage des Messwertes oberhalb oder unterhalb dieses Cut-Off-Wertes der Patient der Gruppe maligne oder benigne zugeordnet.
b) mehrere Indikatorstoffe bzw. Tumormarkerwerte werden gemessen, und anhand eines Entscheidungsbaumes werden mehrere Entscheidungen entsprechend a) vorgenommen, oder sie werden mittels mathematischer Verfahren (z.B. Fuzzifizierung EP 0922266 B1) kombiniert, und mit Hilfe eines artifiziellen Cut-Offwertes (z.B. zwischen 0 und 1) Malignität und Histologie zugeordnet.
c) zwei Indikatorstoffe bzw. Tumormarkerwerte werden gemessen, und aus ihnen ein Verhältnis gebildet. Das Verhältnis ist in stärkerem Maße als ein Tumormarker allein mit dem Vorhandensein einer Tumorerkrankung korreliert. Unter Zuhilfenahme eines an einer Studienpopulation ermittelten Cut-Off-Wertes für dieses Verhältnis wird wieder eine Entscheidung im Sinne von a) gefällt.
d) zusätzlich zu den Indikatorstoffen bzw. Tumormarkern werden Parameter einbezogen (wie z.B. das Alter), die entweder das Krankheitsbild oder die Messeigenschaften der Indikatorstoffe beeinflussen,
e) komplexe Rechenmethoden (z.B. Neuronale Netze), in die Tumormarkerwerte, Indikatorstoffe sowie -werte eingehen, werden angewendet, um Sensitivität und/oder Spezifität der diagnostischen Aussagen zu verbessern.

US2003/0233197-A1 und WO02/095650-A2 offenbaren Verfahren zur Analyse von Genexpressionsdaten mittels Bayesianischer statistischer Analyse zur Extraktion von klinisch relevanter Information.

Die bisherigen Erfindungen zum Prostatakarzinom beschreiben meist die Messung von Tumormarkerwerten, und zugleich die Anwendung der Werte sowie deren Verhältnisse (Ratio) für die Diagnostik:
Als Beispiel ist hier zu nennen die US PS 5,501,983 für Verhältnisse von freiem PSA (fPSA) und komplexiertes PSA (cPSA).

Die dem Stand der Technik nach eingesetzten Lösungen zeichnen sich dadurch aus, dass generell ein großer Überlappungsbereich von an einem Tumor erkrankten und nicht erkrankten Patienten bzgl. o.g. Tumormarker, Indikatorstoffe und -werte bzw. der Rechengrößen vorhanden ist. Eine Lösung ist umso besser, je günstiger der Überlappungsbereich geteilt wird.

Alle bisher bekannten Methoden, die mehrere Tumormarker verwenden, teilen den Überlappungsbereich nicht in optimaler Weise. Weiterhin wird der Überlappungsbereich meist unzureichend (z.B. in Form von Scatterplots) dargestellt oder gar nicht beschrieben. Bei vorliegender, z.B. mathematischer Modellierung ist ein Zusammenhang zur klinischen Diagnostik nur im Sinne einer nicht direkt auf die Patientensituation anwendbaren Wahrscheinlichkeitsaussage darstellbar.

Die Anwendung komplexer Methoden (z.B. neuronaler Netze) führt zu verbesserten Resultaten, ist aber zu sehr den Einzeldaten verhaftet.

Damit weisen alle Lösungen, die auf mehreren Markern, Indikatorstoffen und -werten beruhen, den Nachteil auf, das Risiko, an einem Tumor erkrankt zu sein, nicht adäquat zu beschreiben. Die Lösungen haben entweder einen Messwert oder einen abgeleitetes Wert zum Ergebnis, die zwar mit dem Risiko, an einem Tumor erkrankt zu sein, verknüpft sind (Korrelation), jedoch nicht das Risiko direkt im Sinne des positiven Vorhersagewertes beschreiben.

Erfindungsgemäß wird die Aufgabe dadurch gelöst, dass für die nachfolgenden Verfahrensschritte Indikatorstoffe verwendet werden, die je nach ihrer Art von Tumorzellen produziert, in anderen Körperzellen durch den Tumor hervorgerufen und als tumorunspezifische Stoffe in ihrer Konzentration durch den Tumor verändert werden, die aus einer direkten Stoffanalyse ermittelt worden sind, und dass
(2) die Messwerte der Indikatorstoffe gemäß ihrer Anzahl in einem 2- oder mehrdimensionalen Scatterplot aufgetragen werden und
(3) anhand gespeicherter und diagnostisch gesicherter und mit Hilfe der kernel-density-Methodik mathematisch aufbereiteter Datenblöcke, die im besagten Scatterplot zu einer Dichtekarte führen, einer Dichte zugeordnet werden, die
(4) einem durch den positiven Vorhersagewert dargestellten Risiko entspricht, in einer spezifischen klinischen Anwendungssituation (z.B. Screeningsituation, klinische Eingangsdiagnostik, Nachsorge) an einem Tumor erkrankt zu sein.

Vorteilhaft ist es, wenn es sich bei den Indikatorstoffen zur Diagnose des Prostata-Karzinoms um Tumormarker handelt, die freie oder gebundene Formen oder Molekülanteile des Prostataspezifischen Antigens (PSA) sind. So seien die zweidimensionale Dichtekarten für die Paarungen cPSA - tPSA, fPSA - tPSA oder cPSA - fPSA explizit für die Diagnostik des Prostatakarzinioms aufgeführt. Darüber hinaus ist es möglich, auch Rechengrößen aus Indikatorstoffen als Indikatorwerte einzusetzen. So sind die Größen fPSA/tPSA (Quotient), cPSA/tPSA (Quotient) oder tPSA-fPSA (Differenz) als Indikatorwerte nutzbar, die auf einer - dann zusammen mit einem der oben genannten Parameter - oder beiden Achsen einer 2D-Dichtekarte aufgetragen werden.

Die Erfindung wird anhand von anhand von drei Ausführungsbeispielen näher erläutert. Für jedes Beispiel werden aus Blut oder von anderen Körperflüssigkeiten Makromoleküle als Indikatorstoffe verwendet, die je nach ihrer Art von Tumorzellen produziert, in anderen Körperzellen durch den Tumor hervorgerufen und als tumorunspezifische Stoffe in ihrer Konzentration durch den Tumor verändert werden. Die Messwerte der Indikatorstoffe werden aus einer direkten Stoffanalyse ermittelt. Ferner werden Indikatorwerte einbezogen, die mit dem Krankheitsbild in Zusammenhang stehen (z.B. anatomische Veränderungen) oder die Messeigenschaften der Tumormarker bzw. Indikatorstoffe beeinflussen (z.B. Alter). In den Ausführungsbeispielen werden nachfolgend die weiteren Verfahrensschritte zur Ermittlung der positiven Vorhersage ermittelt.

Die Erfindung ist auch bei der Diagnostik und Differentialdiagnostik anderer Tumore einsetzbar. Beispielsweise können bei der Diagnostik des Bronchialkarzinoms die Tumormarkerpaare CYFRA 21-1 und ProGRP, CYFRA 21-1 und NSE oder Tripel, wobei zusätzlich CEA erfasst wird, verwendet werden. Für die Differentialdiagnostik kleinzelliges vs. nichtkleinzelliges Bronchialkarzinom kann eine Dichtekarte verwendet werden, die ebenfalls auf den Tumormarkerpaaren CYFRA 21-1 und ProGRP oder CYFRA 21-1 und NSE beruht. Für die Differentialdiagnostik innerhalb der nichtkleinzelligen Tumore (Adenokarzinom vs. Plattenepithelkarzinom) ist eine Dichtekarte einsetzbar, die auf den Markerpaaren CYFRA 21-1 und CEA oder SCC und CEA beruht.

Ferner ist der Einsatz der Erfindung nicht auf Tumore beschränkt. Beispielweise können Dichtekarten bei der Diagnostik oder prognostischen Bewertung schweren entzündlichen Geschehens eingesetzt werden. Hier kommen z.B. Entzündungs-Laborparameter wie CRP, IL-6 (oder andere Zytokin-Marker) zum Einsatz. Als zusätzlicher Indikatorwert kann ein Score-Wert benützt werden (z.B. APACHE-Score), der den Patientenzustand zusammenfasst.

Die Anwendung der Dichtekarten ist sowohl in händischer Form (ähnlich Nomogramm) als auch computergestützt umsetzbar.

### Ausführungsbeispiel 1

(1) Die Messwerte der Indikatorstoffe im Blutplasma einer Person werden gemäß dem ersten Verfahrenschritt zusammengefasst. Im Ergebnis erhält man folgende Datenliste:

**Tabelle 1**

| Indikatorstoff | Messwert |
|---|---|
| cPSA | 3 µg/L |
| tPSA | 4,2 µg/L |

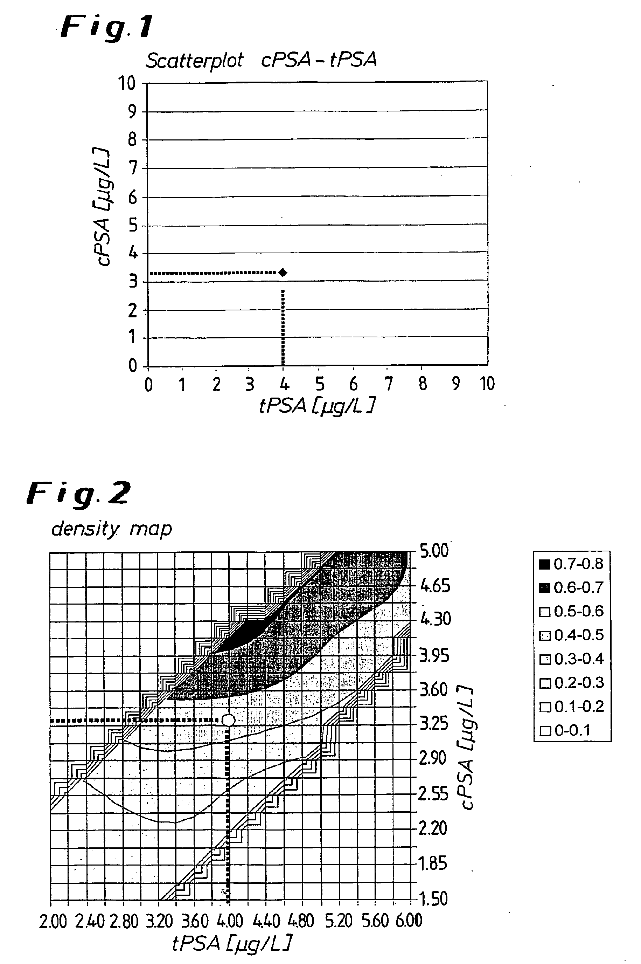

(2) Im zweiten Verfahrensschritt werden die Messwerte in einem Scatterplot aufgetragen. Die Auftragung ist in der Figur 1 gezeigt.
(3) Die Lage im Scatterplot wird in diesem Verfahrenschritt mit den gespeicherten, diagnostisch gesicherten und mit Hilfe der kernel-density-Methodik mathematisch aufbereiteten Datenblöcke, die im besagten Scatterplot zu einer Dichtekarte führen, einer Dichte zugeordnet (siehe Figur 2). Dies ist rechentechnisch oder in Form eines Nomogramms möglich.

Die mathematische Aufbereitung der gespeicherten und diagnostisch gesicherten Datenblöcke erfolgt hierbei in folgender Weise:
a) Auswahl der Datenbasis und Zuordnung zu einer klinischen Anwendungssituation: tPSA und cPSA-Daten aus Anwendungssituation "Klinische Eingangsdiagnostik bei Verdacht auf Prostatakarzinom"
b) Unterteilung des Messbereiches in ein Raster (hier 20x20 Schritte)
c) Berechnung der kernel-densities D_{dis} der Prostatakarzinom-(PCA-)Fälle und D_{nondis} der nicht an Prostatakarzinom erkrankten (nPCA-)Fälle für jeden Rasterpunkt nach z.B. Härdle 2003, wobei ein Gauss-Kernel verwendet wird, und die Liniebreite gemäß der im Zitat angegebenen "Thumbs-Rule" berechnet wird. (Zitat: Härdle W, Simar L: Applied Multivariate Statistical Analysis. Springer-Verlag Berlin Heidelberg 2003, S. 25 ff.)
d) Ermittlung der Quotienten D_{dis}/(D_{dis}+D_{nondis}) für jeden Rasterpunkt.
e) Erstellung der Grafik mit einem geeigneten Computerprogramm

Den Messwerten wird der Dichtebereich 0.5 - 0.6 zugeordnet. (Figur 2)
(4) Dem Dichtebereich entspricht ein positiver Vorhersagerwert von 50-60% für den Patienten, an einem Prostatakarzinom erkrankt zu sein.

### Ausführungsbeispiel 2

(1) Die Messwerte der Indikatorstoffe im Blutplasma einer Person werden gemäß dem ersten Verfahrenschritt zusammengefasst. Im Ergebnis erhält man folgende Datenliste:

**Tabelle 2**

| Indikatorstoff | Messwert |
|---|---|
| tPSA | 6.2 µg/l |
| fPSA/tPSA % | 16% |

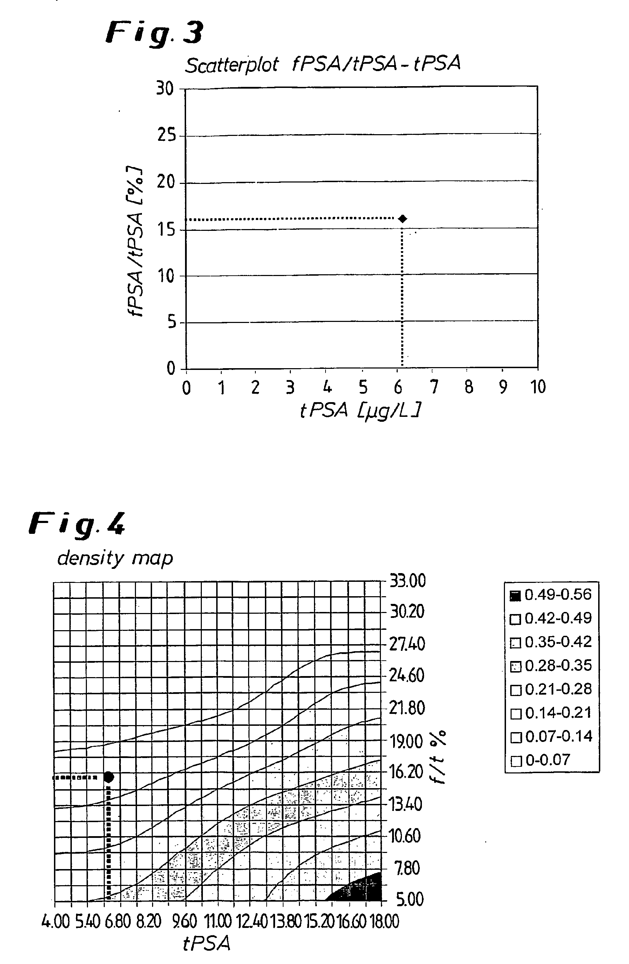

(2) Im zweiten Verfahrensschritt werden die Messwerte in einem Scatterplot aufgetragen. Die Auftragung ist in der Figur 3 gezeigt.
(3) Die Lage im Scatterplot wird in diesem Verfahrenschritt mit den gespeicherten, diagnostisch gesicherten und mit Hilfe der kernel-density-Methodik mathematisch aufbereiteten Datenblöcke, die im besagten Scatterplot zu einer Dichtekarte führen, einer Dichte zugeordnet (siehe Figur 4). Dies ist rechentechnisch oder in Form eines Nomogramms möglich.

Die mathematische Aufbereitung der gespeicherten und diagnostisch gesicherten Datenblöcke erfolgt hierbei in folgender Weise:
a) Auswahl der Datenbasis und Zuordnung zu einer klinischen Anwendungssituation: tPSA und fPSA/tPSA-Daten aus Anwendungssituation "Eingangsdiagnostik Prostatakarzinom"
b) Unterteilung des Messbereiches in ein Raster (hier 20x20 Schritte)
c) Berechnung der kernel-densities D_{dis} der PCA-Fälle und D_{nondis} der nPCA-Fälle für jeden Rasterpunkt nach z.B. Härdle 2003, wobei ein Gauss-Kernel verwendet wird, und die Liniebreite gemäß der im Zitat angegebenen "Thumbs-Rule" berechnet wird. (Zitat: Härdle W, Simar L: Applied Multivariate Statistical Analysis. Springer-Verlag Berlin Heidelberg 2003, S. 25 ff.)
d) Ermittlung der Quotienten D_{dis}/(D_{dis}+D_{nondis}) für jeden Rasterpunkt.
e) Erstellung der Grafik mit einem geeigneten Computerprogramm

Dem Messwerten wird der Dichtebereich 0.07- 0.14 zugeordnet.
(4) Dem Dichtebereich entspricht ein positiver Vorhersagerwert von 7 - 14 % für den Patienten, an einem Prostatakarzinom erkrankt zu sein.

### Ausführungsbeispiel 3

(1) Die Messwerte der Indikatorstoffe im Blutplasma einer Person werden gemäß dem ersten Verfahrenschritt zusammengefasst. Im Ergebnis erhält man folgende Datenliste:

**Tabelle 3**

| Indikatorstoff, Indikatorwert | Messwert |
|---|---|
| tPSA | 3 µg/l |
| cPSA | 4,2 µg/l |
| Prostatavolumen | 62 cm³ |

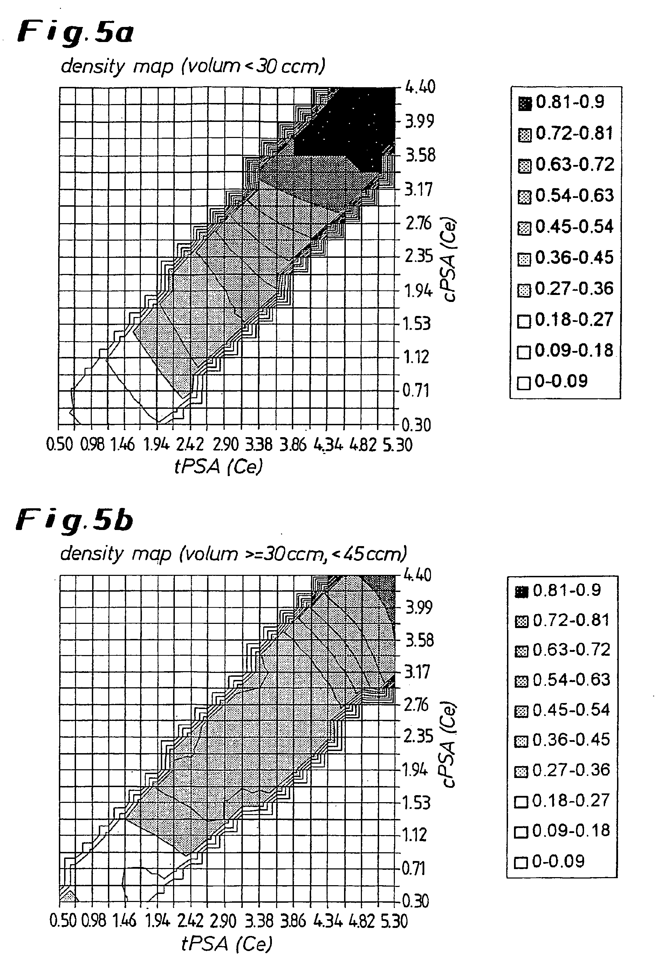

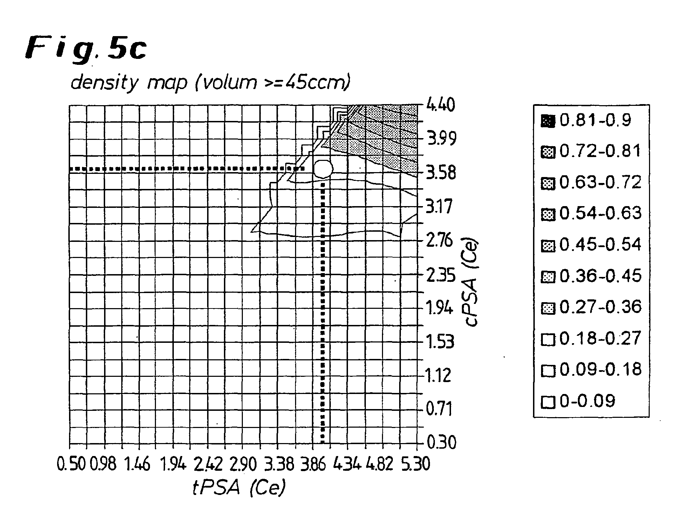

(2) Im zweiten Verfahrensschritt werden die Messwerte in einem 3D-Scatterplot eingetragen. Im vorliegenden Fall ist der 3D-Scatterplot in mehrere Schichten aufgeteilt (Schnitte), die je als 2D-Scatterplot dargestellt sind (Figur 5).
(3) Die Lage im Scatterplot wird in diesem Verfahrenschritt mit den gespeicherten, diagnostisch gesicherten und mit Hilfe der kernel-density-Methodik mathematisch aufbereiteten Datenblöcke, die im besagten Scatterplot zu einer Dichtekarte führen, einer Dichte zugeordnet (siehe Figur 6). Dies ist rechentechnisch oder in Form eines Nomogramms möglich.

Die mathematische Aufbereitung der gespeicherten und diagnostisch gesicherten Datenblöcke erfolgt hierbei in folgender Weise:
a) Auswahl der Datenbasis und Zuordnung zu einer klinischen Anwendungssituation: tPSA und cPSA-Daten aus Anwendungssituation "Klinische Eingangsdiagnostik bei Verdacht auf Prostatakarzinom"
b) Unterteilung des Messbereiches in ein Raster (hier 20x20x3 Schritte)
c) Berechnung der kernel-densities D_{dis} der PCA-Fälle und D_{nondis} der nPCA-Fälle für jeden Rasterpunkt nach z.B. Härdle 2003, wobei ein Gauss-Kernel verwendet wird, und die Liniebreite gemäß der im Zitat angegebenen "Thumbs-Rule" berechnet wird. (Zitat: Härdle W, Simar L: Applied Multivariate Statistical Analysis. Springer-Verlag Berlin Heidelberg 2003, S. 25 ff.)
d) Ermittlung der Quotienten D_{dis}/(D_{dis}+D_{nondis}) für jeden Rasterpunkt.
e) Erstellung der 3D-Grafik oder der 2D-Grafikschichten Grafikschichten mit einem geeigneten Computerprogramm

Dem Messwerten wird auf der zum Prostatavolumen zugehörigen Dichteschicht >45 ccm der Dichtebereich 0.18 - 0.27 zugeordnet.
(4) Dem Dichtebereich entspricht ein positiver Vorhersagerwert von 18-27% für den Patienten, an einem Prostatakarzinom erkrankt zu sein (bei gleichen Tumormarkerwerten deutlich geringer als im Ausführungsbeispiel 1).

## Patentansprüche

1. Verfahren zur patientenspezifischen Zuordnung eines Risikos, an Prostatakarzinom erkrankt zu sein, anhand von n Indikatorstoffen aus Körperflüssigkeiten, **dadurch gekennzeichnet, dass**
a) n > 1 ist,
b) die Messwerte x_{i, pat} des Patienten von n Indikatorstoffen (i=1, ..,n) in einem n- dimensionalen Koordinatensystem aufgetragen werden, so dass sich der Punkt (x_{1, pat,} X_{2,pat}, ..., X_{n,pat}) ergibt,
c) in dem Koordinatensystem innerhalb des durch die Wertebereiche der einzelnen Indikatorstoffe aufgespannten Raumes Kurven oder Flächen berechnet werden, die Funktionen g für den Indikatorstoff k in Abhängigkeit von den restlichen n-1 Indikatorstoffen (1,.. , k-1, k+1, ..., n) darstellen und sich dadurch auszeichnen, dass sie Kurven oder Flächen gleicher Funktionswerte y einer Funktion y = f(x₁, x₂, ..., xₙ) sind, wobei sich die Funktion y aus dem Quotienten aus den mittels kernel-density-Methodik berechneten Dichteflächen für (i) erkrankte Patienten und (ii) der Summe aus erkrankten und nichterkrankten Patienten einer in dem n-dimensionalen. Koordinatensystem aufgetragenen Studienpopulation ergibt,
d) aus der Gesamtheit der berechneten Kurven oder Flächen eine Subgruppe mit p Kurven oder Flächen ausgewählt wird,
e) dem Punkt (x₁, x₂, ..., xₙ) ein Wert zwischen den durch die beiden nächst- gelegenen Kurven oder Flächen der Subgruppe repräsentierten Funktionswerten zugeordnet wird und dieser zugeordnete Wert einem bestimmten Risiko an einer Krankheit erkrankt zu sein entspricht;
**dadurch gekennzeichnet, dass** die Indikatorstoffe cPSA oder tPSA oder fPSA oder andere molekularen Formen von PSA (z. B. BPSA, proPSA, intact-PSA, PSA-API, PSA-ACT, PSA-alpha-2M) oder Rechengrössen aus beliebigen Kombinationen dieser molekularen PSA-Formen aus dem Blutserum verwendet werden.

2. Verfahren nach Anspruch 1, **dadurch gekennzeichnet, dass** der zugeordnete Wert in Schritt e) der Mittelwert zwischen den Funktionswerten ist.

3. Verfahren nach Anspruch 1 oder 2, **dadurch gekennzeichnet, dass** die Studienpopulation in Schritt c) eine solche ist, wie sie üblicherweise zur Festlegung von Cut-Off-Werten für die einzelnen Indikatorwerte zur Diagnostik der Krankheit verwendet wird.

4. Verfahren nach einem der Ansprüche 1 bis 3, **dadurch gekennzeichnet, dass** zusätzlich weitere Glättungsverfahren, Wichtungsverfahren sowie Verfahren zur Ausreisserkontrolle bei der Berechnung nach der kernel-density-Methodik in Schritt c) zur Anwendung kommen.

5. Verfahren nach einem der Ansprüche 1 bis 4, **dadurch gekennzeichnet, dass** statt der Indikatorstoffe andere Parameter, die spezifisch mit der Tumorerkrankung verbunden sind, in eine gleichartige die Auswertung einbezogen werden, wobei jedoch mindestens 'ein Indikatorstoff in die Analyse eingeht.

## Claims

1. Method for patient-specific assignment of a risk of having contracted prostate carcinoma on the basis of n indicator substances from body fluids, **characterised in that**
a) n is >1,
b) the measured values x_{i, pat} of the patient for n indicator substances (i=1, ...,n) are plotted in an n-dimensional coordinate system so as to yield the point (x_{1, pat,} x_{2,pat,} ..., x_{n,pat}),
c) curves or areas in the coordinate system within the space spanned by the value ranges of the individual indicator substances are calculated which represent functions g for the indicator substance k as a function of the remaining n-1 indicator substances (1,..., k-1, k+1, ..., n) and are distinguished **in that** they are curves or areas of identical function values y of a function y = f(x₁, x₂, ..., xₙ), with the function y being obtained from the quotient of the density areas calculated using the kernel density method for (i) diseased patients and (ii) the sum total of diseased and non-diseased patients in a study population plotted in the n-dimensional coordinate system,
d) a subgroup containing p curves or areas is selected from the totality of calculated curves or areas,
e) the point (x₁, x₂, ..., xₙ) is assigned a value between the function values represented by the two next adjacent curves or areas of the subgroup and said assigned value corresponds to a specific risk of having contracted a disease;
**characterised in that** the indicator substances cPSA or tPSA or fPSA or other molecular forms of PSA (e.g. BPSA, proPSA, intact-PSA, PSA-API, PSA-ACT, PSA-alpha-2M) or computational operands from arbitrary combinations of said molecular PSA forms from the serum are used.

2. Method according to claim 1, **characterised in that** the assigned value in step e) is the mean value between the function values.

3. Method according to claim 1 or 2, **characterised in that** the study population in step c) is such as is typically used for setting cut-off values for the individual indicator values for diagnosing the disease.

4. Method according to one of claims 1 to 3, **characterised in that** further smoothing methods, weighting methods and outlier control methods are used in addition for the calculation according to the kernel density method in step c).

5. Method according to one of claims 1 to 4, **characterised in that** instead of the indicator substances other parameters that are specifically associated with the tumorous disease are incorporated in a similar assessment, in which case, however, at least one indicator substance is included in the analysis.

## Revendications

1. Procédé d'affectation spécifique à un patient d'un risque d'être atteint d'un carcinome de la prostate au moyen de n substances indicatrices de liquides corporels, **caractérisé en ce que**
a) n > 1,
b) les valeurs x_{1, pat} de mesure du patient de n substances (i=1, ..., n) indicatrices sont portées dans un système de coordonnées à n-dimensions, de manière à obtenir le point (X_{1,pat,} X_{2,pat,} ...X_{n,pat}),
c) on calcule, dans le système de coordonnées, dans l'espace délimité par les zones de valeur des diverses substances indicatrices, des courbes ou des surfaces qui représentent des fonctions g pour la substance k indicatrice en fonction des n-1 substances (1..., k-1, k+1, ...n) indicatrices restantes et qui se caractérisent par le fait que ce sont des courbes ou des surfaces de même valeur y d'une fonction y = f(x₁, x_{2,} ..., xₙ), la fonction y étant obtenue par le quotient de surface de densité calculé au moyen de la méthode kernel-density pour (i) des patients malades et (ii) la somme de patients malades et non malades d'une population étudiée portée dans le système de coordonnées à n-dimensions,
d) on sélectionne un sous-groupe ayant p courbes ou surfaces dans l'ensemble des courbes ou surfaces calculées,
e) on affecte aux points (x_{1,} x_{2,} ...xₙ) une valeur entre les valeurs de fonction représentées par les deux courbes ou surfaces les plus proches du sous-groupe et cette valeur affectée correspond à un risque déterminé de souffrir d'une maladie ;
**caractérisé en ce que**
on utilise les substances cPSA ou tPSA ou fPSA indicatrices ou d'autres formes moléculaires de PSA par exemple (BPSA, proPSA, intact-PSA, PSA-API, PSA-ACT, PSA-alpha-2M) ou des grandeurs calculées de n'importe quelle combinaison de ces formes moléculaires de PSA du sérum sanguin.

2. Procédé suivant la revendication 1,
**caractérisé en ce que**
la valeur affectée au stade e) est la moyenne entre les valeurs de fonction.

3. Procédé suivant la revendication 1 ou 2,
**caractérisé en ce que**
la population étudiée au stade c) est telle qu'elle est utilisée habituellement pour la détermination de valeurs de cut-off pour les diverses valeurs indicatrices en vue du diagnostic de la maladie.

4. Procédé suivant l'une des revendications 1 à 3,
**caractérisé en ce que**
on applique, en outre, d'autres procédés de lissage, procédés de pondération, ainsi que des procédés de contrôle d'observation extrême aberrante dans le calcul suivant la méthode kernel-density au stade c).

5. Procédé suivant l'une des revendications 1 à 4,
**caractérisé en ce que**
au lieu des substances indicatrices, on inclut, dans une évaluation de même type, d'autres paramètres, qui sont liés spécifiquement à la maladie tumorale, au moins une substance indicatrice entrant toutefois dans l'analyse.
